# EUROPEAN PATENT APPLICATION

(11) **EP 4 393 378 A2**
(43) Date of publication of application: **03.07.2024**
(21) Application number: 23219785.5
(22) Date of filing: 22.12.2023
(51) Int. Cl.: A61B 5/00, A61B 17/00, A61B 18/00, A61B 18/14, A61B 34/20

(54) **SYSTEMS AND METHODS FOR CYLINDRICAL CAGE MAPPING AND ABLATION CATHETERS COMPRISING FLEXIBLE CIRCUITS**

(30) Priority: 29.12.2022 US 202263477790 P; 15.11.2023 US 202318510040
(71) Applicant: Biosense Webster (Israel) Ltd., 2066717 Yokneam (IL)
(72) Inventor: RODRIGUEZ SOTO, Juan, Irvine, 92618 (US); ABBAS, Mohammad, Irvine, 92618 (US); EBRAHIMI, Babak, Irvine, 92618 (US); VAN NIEKERK, Pieter Emmelius, Irvine, 92618 (US); BASU, Shubhayu, Irvine, 92618 (US)
(74) Representative: Carpmaels & Ransford LLP

(57) **Abstract**

The disclosed technology includes basket catheters comprising a generally cylindrical structure having electrodes attached thereto. The disclosed technology can include a one-piece flex circuit comprising a proximal end and extending along a longitudinal axis to a distal end. The proximal end diverges into a plurality of first arms, each of the first arms divides into second and third arms that are connected to respective fourth arms proximate the distal end to define an elongated shape disposed about the longitudinal axis. The disclosed technology can include a structural unit for an end effector having a proximal hub, a distal hub, and a plurality of spines forming a generally cylindrical structure. The spines can comprise a plurality of first members attached to the proximal hub that diverge into a plurality of second and third members connected to respective fourth members proximate the distal hub.

## Description

### CROSS REFERENCE TO RELATED APPLICATIONS

This application claims the benefit of priority under 35 U.S.C. § 119 to prior filed U.S. Provisional Patent Application No. 63/477,790, filed December 29, 2022, the entire contents of which is hereby incorporated by reference as if set forth in full herein.

### FIELD

The present invention relates generally to medical devices, and in particular to basket catheters comprising a generally cylindrical structure having electrodes attached thereto.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. Nos. 2021/0169550A1 (now U.S. Patent No. 11,660,135), 2021/0169567A1, 2021/0169568A1, 2021/0161592A1 (now U.S. Patent No. 11,540,877), 2021/0196372A1, 2021/0177503A1, and 2021/0186604A1 (now U.S. Patent No. 11,707,320), each of which are incorporated herein by reference and attached in the appendix to priority application U.S. 63/477,790.

Basket catheters are commonly used for mapping or ablating cardiac tissue. For example, basket catheters are described in U.S. Pat. Nos. 5,772,590, 6,748,255 and 6,973,340, each of which are incorporated herein by reference and attached in the appendix to priority application U.S. 63/477,790. Basket catheters generally include a plurality of spines attached to the distal end of the catheter and configured to form a generally spherical shape. Each spine typically has at least one electrode attached thereto configured for mapping or ablation of tissue. Manufacturing basket catheters can be a difficult and expensive process due to the small size and complex geometry. In particular, attaching electrodes to the spines can be difficult, time consuming, and expensive. Accordingly, there is a need in the art for improved methods of manufacturing basket catheters.

An example mapping and ablation procedure with a basket catheter can include bringing a basket catheter into contact with tissue (e.g., cardiac tissue) and detecting electrical signals at the tissue to map the electrical signals across the tissue and/or delivering electrical energy to the electrodes to ablate the tissue. Because of the spherical configuration of the basket catheter, spherical basket catheters tend to be well-suited for ablating rounded geometries, such as the pulmonary vein (e.g., for pulmonary vein isolation), but less effective at mapping electrical pulses across planar tissue surface. Therefore, in some instances, multiple catheters must be used to map and/or ablate tissue in a heart. As will be appreciated, using multiple catheters requires the physician to insert and manipulate the multiple catheters, resulting in longer surgical times. Thus, there is a need in the art for catheter designs capable of performing ablation and mapping of planar tissue as well as rounded tissues, such as a pulmonary vein.

### SUMMARY

There is provided in an example of the present disclosure a one-piece flex circuit comprising a proximal end and extending along a longitudinal axis to a distal end. The proximal end can diverge into a plurality of first arms. Each of the first arms can divide into second and third arms. Each of the second and third arms can be connected to respective fourth arms proximate the distal end to define an elongated shape disposed about the longitudinal axis.

The disclosed technology can include a structural unit for an end effector comprising a proximal hub, a distal hub positioned a distance along a longitudinal axis away from the proximal hub, a plurality of spines extending between the proximal hub and the distal hub and forming a generally cylindrical structure. The plurality of spines can comprise a plurality of first members attached to the proximal hub. The plurality of first members can diverge into a plurality of second and third members. Each of the second and third members can be connected to respective fourth members proximate the distal hub.

The disclosed technology can include a medical probe comprising a structural unit for an end effector. The structural unit can comprise a proximal hub, a distal hub positioned a distance along a longitudinal axis away from the proximal hub, and a plurality of spines extending between the proximal hub and the distal hub and forming a generally cylindrical structure. The plurality of spines can comprise a plurality of first members attached to the proximal hub. The plurality of first members can diverge into a plurality of second and third members. Each of the second and third members can be connected to respective fourth members proximate the distal hub.

A flexible circuit can be coupled to the structural unit. The flexible circuit can comprise a proximal end and a distal end. The proximal end can diverge into a plurality of first arms. Each of the first arms can divide into second and third arms. Each of the second and third arms can be connected to respective fourth arms proximate the distal end.

The disclosed technology can include a method of constructing a medical probe. The method can comprise forming a plurality of spines. The plurality of spines can form a substantially cylindrical shape and comprise a plurality of first members attached to a proximal hub. The plurality of first members can diverge into a plurality of second and third members. Each of the second and third members can be connected to respective fourth members proximate a distal hub.

The method can include forming a flexible circuit including a plurality of electrodes, coupling the flexible circuit to the plurality of spines to form a cylindrical structure, and attaching the cylindrical structure to a distal end of a tubular shaft.

### BRIEF DESCRIPTION OF THE DRAWINGS

The present disclosure will be more fully understood from the following detailed description of the examples thereof, taken together with the drawings in which:
FIG. 1 is a schematic pictorial illustration of a medical system including a medical probe whose distal end includes a basket assembly with electrodes, in accordance with an example of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in an expanded form, in accordance with the disclosed technology;
FIG. 2C is a schematic pictorial illustration showing a side view of a medical probe in a collapsed form, in accordance with the disclosed technology;
FIG. 2D is a schematic pictorial illustration showing another side view of a medical probe detailing features of a spine of the medical probe, in accordance with the disclosed technology;
FIG. 2E is a schematic pictorial illustration showing a distal end of a medical probe in a collapsed form, in accordance with the disclosed technology;
FIG. 2F is a schematic pictorial illustration of a top view of a medical probe in an expanded form, in accordance with the disclosed technology;
FIG. 3A is a schematic pictorial illustration of a side view of another medical probe in an expanded form, in accordance with the disclosed technology;
FIG. 3B is a schematic pictorial illustration of a distal end a medical probe in a collapsed form, in accordance with the disclosed technology;
FIG. 3C is a schematic pictorial illustration of a top view of a medical probe in an expanded form, in accordance with the disclosed technology;
FIG. 4A is a schematic pictorial illustration of front view of a flexible circuit, in accordance with the disclosed technology;
FIG. 4B is a detail view of a front side of an electrode of a flexible circuit, in accordance with the disclosed technology;
FIG. 4C is a side of an electrode of a flexible circuit, in accordance with the disclosed technology;
FIG. 4D is a detail view of contacts of a flexible circuit, in accordance with the disclosed technology;
FIG. 5A is a schematic pictorial illustration of a front view of a flexible circuit, in accordance with the disclosed technology;
FIG. 5B is a detail view of an electrode of a flexible circuit, in accordance with the disclosed technology;
FIG. 5C is a side view of an electrode of a flexible circuit, in accordance with the disclosed technology;
FIG. 6A is a schematic pictorial illustration of a flexible circuit in a collapsed configuration, in accordance with the disclosed technology;
FIG. 6B is a schematic pictorial illustration of a flexible circuit in an expanded configuration, in accordance with the disclosed technology; and
FIG. 7 is a flowchart of a method of manufacturing a medical probe, in accordance with the disclosed technology.

### DETAILED DESCRIPTION

As will become apparent throughout this disclosure, the disclosed technology includes several advantages over the prior art. The disclosed technology can include an expandable basket assembly having a generally cylindrical shape. The expandable basket assembly can have a plurality of electrodes attached thereto that can be configured for mapping or ablation of tissue. By including an expandable basket assembly having a generally cylindrical shape, the disclosed technology can be well suited for mapping or ablating generally planar tissue as well as generally circularly-shaped tissue such as a pulmonary vein. For instance, the generally cylindrical shape can have an approximately planar end having electrodes disposed thereon for mapping or ablating generally planar tissue. The generally cylindrical shape can further include electrodes disposed around an outer circumferential surface that can be configured to mapping or ablating generally circularly-shaped tissue.

The disclosed technology can include a flexible circuit that can be attached to a generally cylindrical basket. The flexible circuit can include a plurality of electrodes configured for mapping and/or ablating tissue. In this way, the flexible circuit can be made separate from the cylindrical basket and then be attached thereto, thereby simplifying the process of manufacturing the expandable basket assembly.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including , but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing thermal energy such as RF ablation or cryoablation, or non-thermal energy, such as irreversible electroporation (IRE), referred throughout this disclosure interchangeably as pulsed electric field (PEF) and pulsed field ablation (PFA). IRE ablation can include monophasic or biphasic pulses. Furthermore, the disclosed technology can include reversible electroporation.

Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

Unless otherwise specified, the use of the ordinal adjectives "first," "second," "third," etc., to describe a common object or related objects, merely indicate that different instances of like objects are being referred to, and are not intended to imply that the objects so described should be in a given sequence, either temporally, spatially, in ranking, or in any other manner.

The present disclosure is related to systems, methods or uses and devices which utilize end effectors including cylindrical basket catheters having a flexible circuit comprising electrodes attached thereto. Example systems, methods, and devices of the present disclosure may be particularly suited for mapping and ablation of cardiac tissue to treat cardiac arrhythmias. Ablative procedures incorporating such example catheters can be visualized using fluoroscopy.

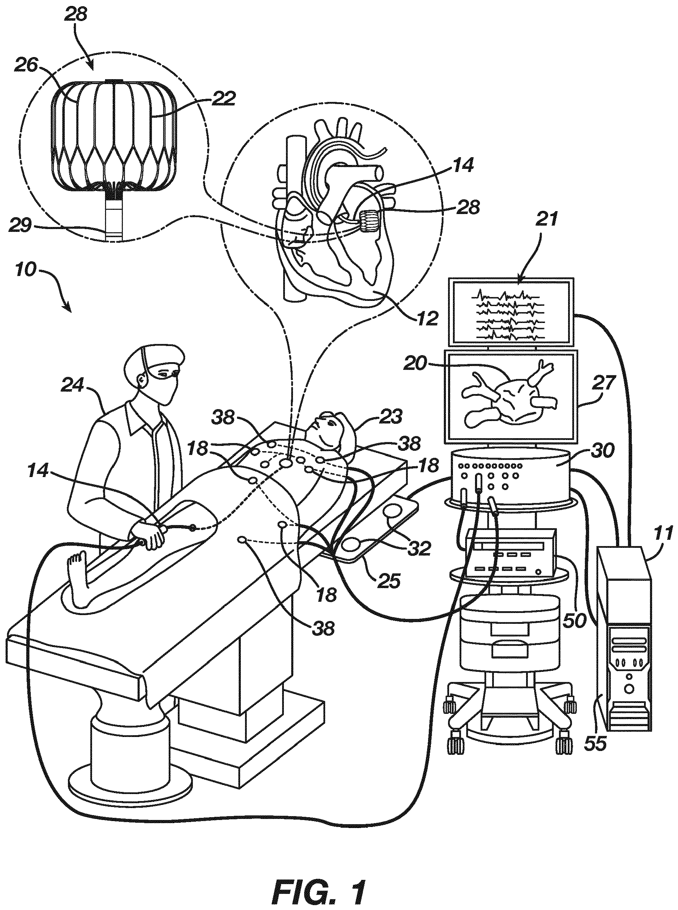

Reference is made to FIG. 1, showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip (basket assembly 28) of catheter 14 comprising a medical probe 16 into contact with the heart wall at or near the pulmonary vein for sensing a target site in heart 12. For ablation, physician 24 would similarly bring a distal end of an ablation catheter comprising medical probe 16 to a target site for ablating.

Medical probe 16 is an exemplary probe that includes one and preferably multiple electrodes 26 optionally distributed over a plurality of spines 22 at basket assembly 28 and configured to sense the IEGM signals. Medical probe 16 may additionally include a position sensor 29 embedded in or near basket assembly 28 for tracking position and orientation of basket assembly 28. Optionally and preferably, position sensor 29 is a magnetic based position sensor including three magnetic coils for sensing three-dimensional (3D) position and orientation. Position sensor 29 can be conventional coiled wire sensors, flat PCB based sensors, or deformable electromagnetic loop sensors. Although not depicted, position sensor 29 can alternatively be positioned on the plurality of spines 22 or designed into individual spines of the plurality of spines 22. In some embodiments, individual spines of the plurality of spines 22 can be insulated and act as a position sensor.

In some embodiments, medical probe 16 can include a deformable electromagnetic loop sensor Examples of various systems and methods for deformable electromagnetic loop sensors are presented in U.S. Patent No.'s 11,304,642 and 10,330,742, and U.S. Patent Publications 2018/0344202A1 and 2020/0155224A1, the entireties of each of which are incorporated herein by reference and attached in the appendix to priority application U.S. 63/477,790.

Magnetic based position sensor 29 may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the appendix to priority application U.S. 63/477,790.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in U.S. Patent Nos. 7,536,218; 7,756,576; 7,848,787; 7,869,865; and 8,456,182, each of which are incorporated herein by reference and attached in the appendix to priority application U.S. 63/477,790.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618 USA.

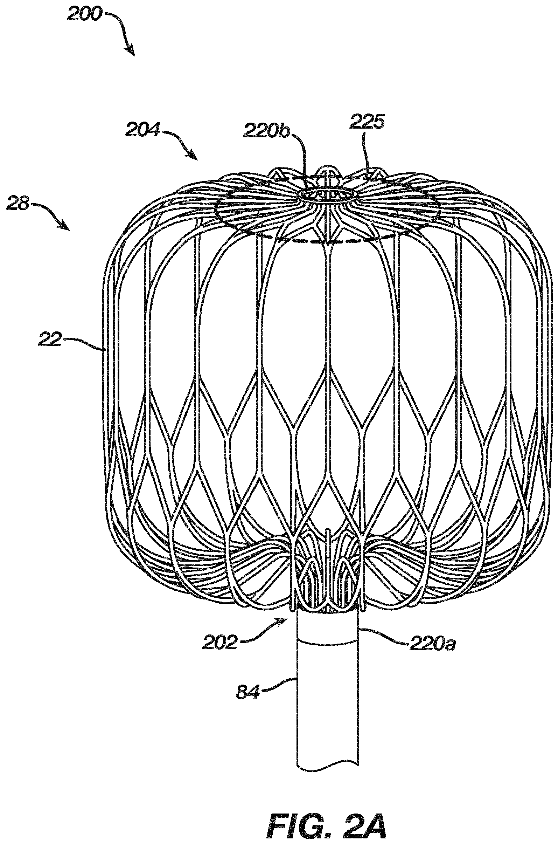

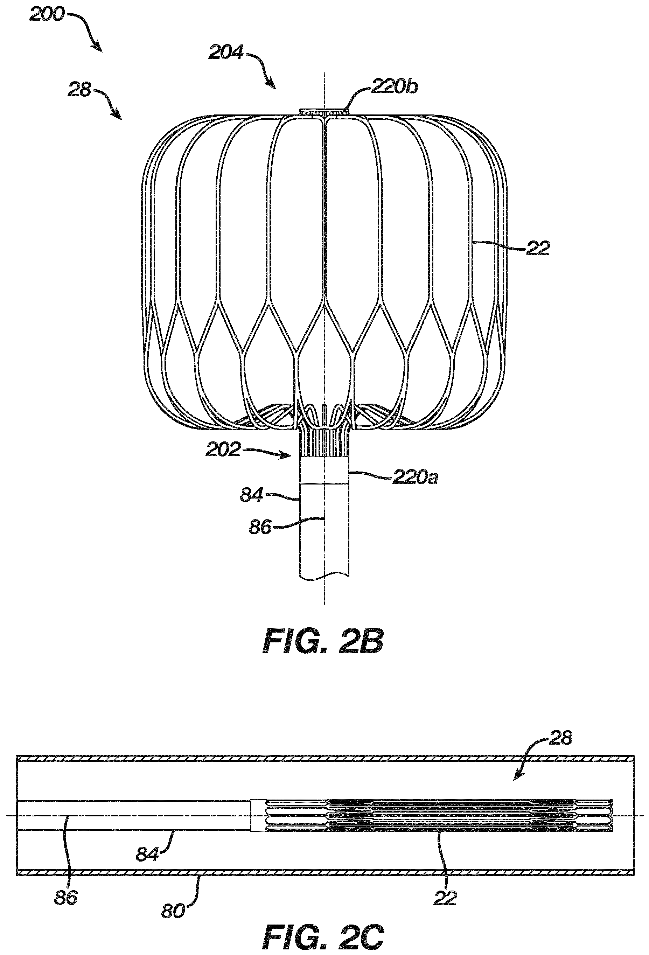

FIG. 2A is a schematic pictorial illustration showing a perspective view of a medical probe 200 in an expanded form while FIG. 2B is a schematic pictorial illustration showing a side view of the medical probe 200 in the expanded form, in accordance with the disclosed technology. The medical probe 200 can be in an expanded form when unconstrained, such as by being advanced out of a delivery sheath 80 by a tubular shaft 84. FIG. 2C, on the other hand, is a schematic pictorial illustration showing a side view of the medical probe 200 in a collapsed form, such as by being retracted into the delivery sheath 80. In the expanded form (FIGs. 2A and 2B), the spines 22 bow radially outwardly along a longitudinal axis 86 and in the collapsed form (FIG. 2C) the spines are constrained generally along the longitudinal axis 86 of tubular shaft 84.

As shown in FIGs. 2A and 2B, the medical probe 200 can include a basket assembly 28 comprising a plurality of flexible spines 22 that are formed at the end of a tubular shaft 84 and are connected at both ends. During a medical procedure, physician 24 can deploy basket assembly 28 by extending tubular shaft 84 from delivery sheath 80 (FIG. 2C) causing the basket assembly 28 to exit the delivery sheath 80 and transition to the expanded form.

As shown in FIGs. 2A and 2B, the basket assembly 28 can include a proximal end 202 and a distal end 204 and form a generally cylindrical shape. The spines 22 can extend between a proximal hub 220a and a distal hub 220b to which the spines 22 can be connected. The distal end 204 can include a generally planar portion 225. As will be described in greater detail herein, the basket assembly 28 can include a plurality of electrodes 26 (not shown in FIGs. 2A-2C) formed into a flexible circuit that can be attached to the basket assembly 28. Due to the cylindrical shape of the basket assembly 28, the medical probe 200 described herein can be configured for mapping and/or ablation of both planar tissue (e.g., generally planar surfaces of a heart) and rounded tissue locations (e.g., a pulmonary vein). In this way, the medical probe 200 can be configured to perform mapping and/or ablation of multiple surfaces of an organ in a single procedure without requiring the use of multiple catheters having different end effector shapes. The planar portion 225, for example, can have a plurality of electrodes 26 disposed thereon configured for mapping and/or ablation of generally planar tissue and the sides of the cylindrical structure of the basket assembly 28 can include a plurality of electrodes 26 disposed thereon configured for mapping and/or ablation of generally rounded tissue location.

The basket assembly 28 can be formed from a planar sheet of biocompatible material or a cylindrical stock of biocompatible material. For example, the spines 22 can be cut into the biocompatible material and then the basket assembly 28 can be formed into the cylindrical shape by heat setting or otherwise causing the spines 22 to retain the cylindrical shape. The biocompatible material can be any suitable type of biocompatible material including, but not limited to, nitinol, stainless steel, cobalt chromium, polymer material, or other suitable materials.

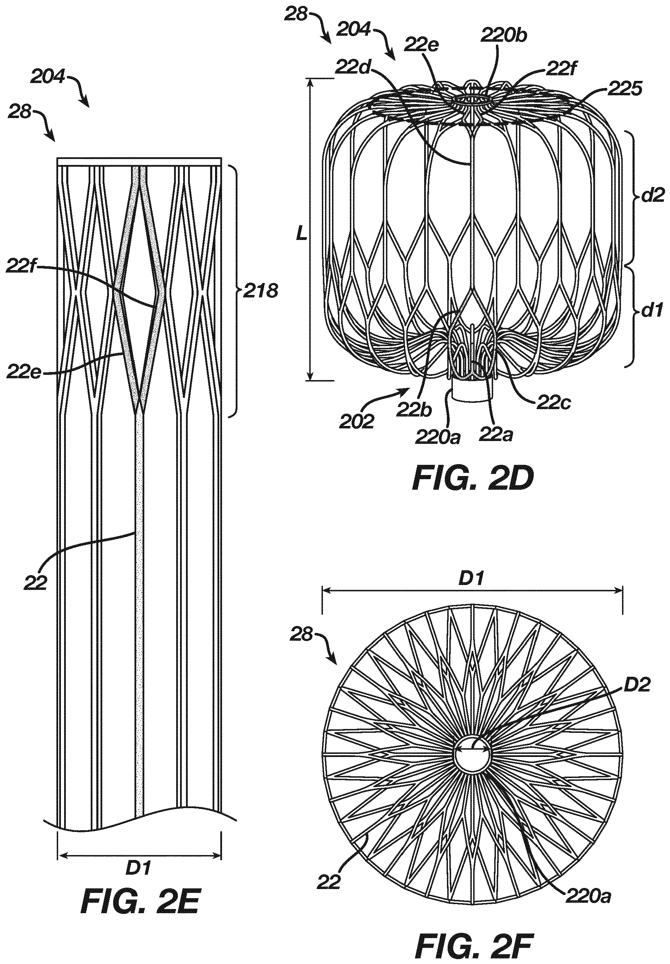

FIG. 2D is a schematic pictorial illustration showing another side view of a basket assembly 28 of medical probe 200 detailing features of a spine 22 of the medical probe 200, in accordance with the disclosed technology. FIG. 2E is a schematic pictorial illustration showing a distal end of a basket assembly 28 of medical probe 200 in a collapsed form and FIG. 2F is a schematic pictorial illustration of a top view of a basket assembly 28 of medical probe 200 in an expanded form, in accordance with the disclosed technology.

As shown in FIG. 2D, the basket assembly 28, which can act as a structural unit configured to support electrodes 26 (described in greater detail herein), can include a generally cylindrical shape formed by spines 22 extending between the proximal hub 220a and the distal hub 220b a length L. As shown in FIG. 2F, the basket assembly 28 can include an outer diameter D 1 and an inner diameter D2. As will be appreciated, the length L, the outer diameter D1, and the inner diameter D2 can be sized as appropriate for the particular application.

As shown in FIG. 2D, 2E, and 2F, each spine 22 can be broken down into individual members 22a-22f to as it extends between the proximal hub 220a and the distal hub 220b. For example, the spine 22 can include a first member 22a that is attached the proximal hub 220a at a first end and diverges into a second member 22b and a third member 22c at a first location along the spine 22 distal from the proximal hub 220a. As shown in FIG. 2D, the second member 22b and the third member 22c can extend a first distance d1 along the length L of the basket assembly 28. The second member 22b and the third member 22c can converge together to a fourth member 22d at a second location along the spine 22 distal from the first location and proximate the distal hub 220b. The fourth member 22d can extend a second distance d2 along the length L of the basket assembly 28. The spine 22 can further diverge into a fifth member 22e and a sixth member 22f at a third location distal from the second location and proximate the distal hub 220b. The fifth member 22e and the sixth member 22f can converge and be attached to the distal hub 220b at a distal end of the spine 22. As will be appreciated, all of the members 22a-22f can be a continuous piece of biocompatible material or all of the members 22a-22f can be individual members that are connected together to form the spine 22.

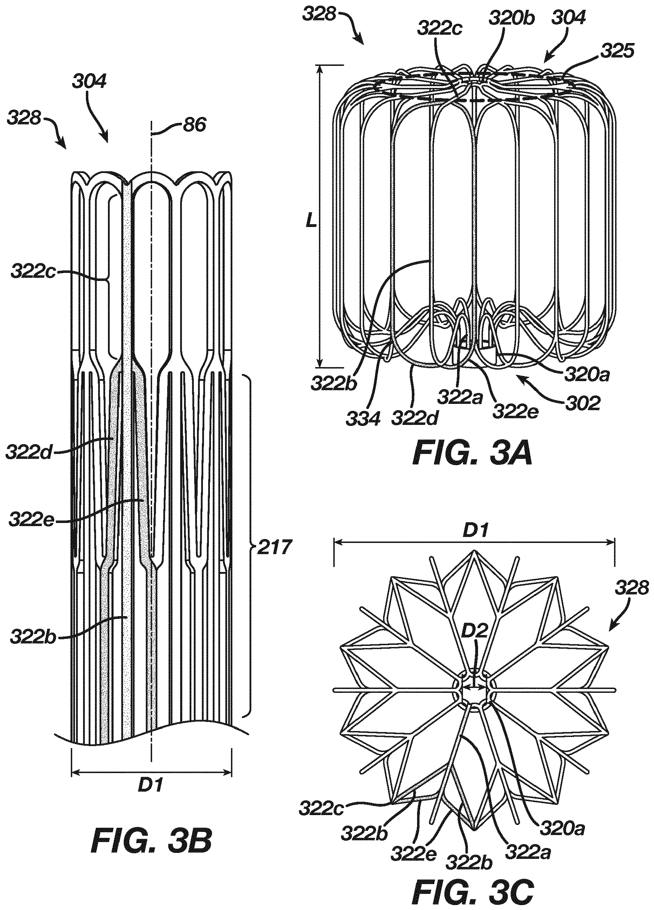

FIG. 3A is a schematic pictorial illustration of a side view of another basket assembly 328 in an expanded form, FIG. 3B is a schematic pictorial illustration of a distal end another basket assembly 328 in a collapsed form, and FIG. 3C is a schematic pictorial illustration of a top view of a basket assembly 328 in an expanded form, in accordance with the disclosed technology. As shown in FIGs. 3A-3C, the basket assembly 328 can form a generally cylindrical shape extending between a proximal end 320 and a distal end 304 like the basket assembly 28 previously described. Similarly, the basket assembly 328 can have a generally planar portion 325 at a distal end 304 of the basket assembly 328.

As shown in FIG. 3A, the basket assembly 328, which can act as a structural unit configured to support electrodes 26 (described in greater detail herein), can include a generally cylindrical shape formed by spines 322 extending between the proximal hub 320a and the distal hub 320b a length L. As shown in FIG. 3C, the basket assembly 328 can include an outer diameter D 1 and an inner diameter D2. As will be appreciated, the length L, the outer diameter D1, and the inner diameter D2 can be sized as appropriate for the particular application.

As shown in FIGs. 3A-3C, each spine 322 can be broken down into individual members 322a-322e to as it extends between the proximal hub 320a and the distal hub 320b. For example, the spine 322 can include a first member 322a that is attached the proximal hub 320a at a first end and a second member 322b at a second end. The second member 322b can extend substantially along the entire edge of the cylindrical structure. The second member 322b can be attached to a third member 322c at a distal end of the second member 322b. The third member 322c can extend from the second member 322b to the distal hub 320b and be attached to the distal hub 320b. As will be appreciated, the first member 322a, the second member 322b, and the third member 322c can be a single continuous spine extending between the proximal hub 320a and the distal hub 320b.

The basket assembly 328 can further include a fourth member 322d and a fifth member 322e that can diverge from the first member 322a at a point where the first member 322a and the second member 322b are connected. The fourth member 322d and the fifth member 322e can extend along the length of the basket assembly 328 distally and converge together at a point where the second member 322b and third member 322c are attached to each other. As will be appreciated, all of the members 322a-322e can be a continuous piece of biocompatible material or all of the members 322a-322e can be individual members that are connected together to form the spine 322. In some examples, neighboring fourth members 322d and fifth members 322e can connect to each other at a connection point 334 to connect the basket assembly 328 together.

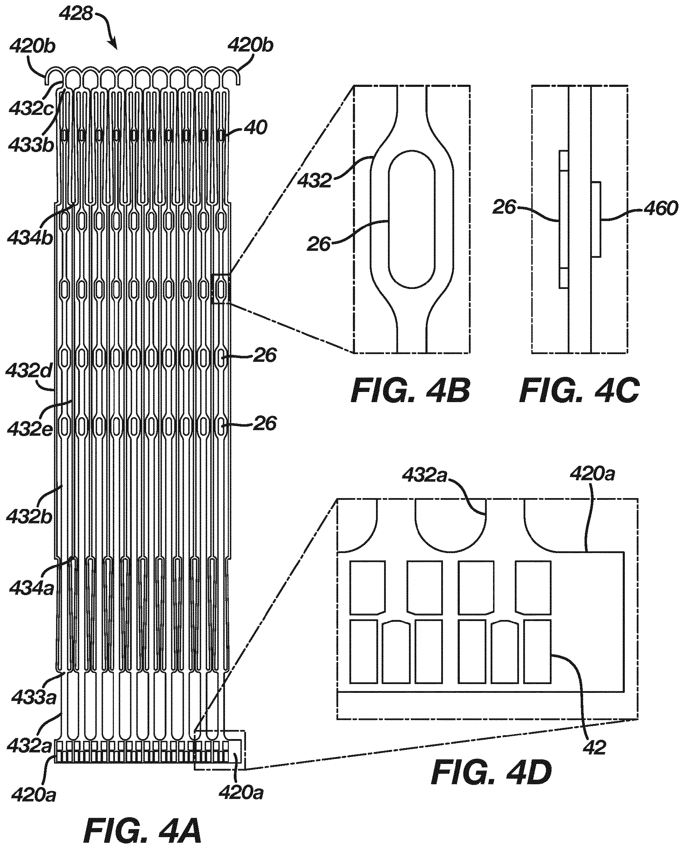

As described herein, the disclosed technology can include a flexible circuit 428 that can be attached to the basket assembly 28 (or basket assembly 328) to form the medical probe. In addition, flexible circuit 428 can be directly fabricated onto the basket assembly 28. FIGs. 4A-6B show various examples of such flexible circuits. Turning now to FIGs. 4A-4D, a flexible circuit 428 is shown and described. As shown, the flexible circuit 428 can include a plurality of arms 432 that can be configured for alignment with the spines 22 (or spines 322) previously described. The arms 432 can each extend between a proximal connector 420a at a proximal end and a distal connector 420b at a distal end. the proximal connector 420a can be configured for connecting the flexible circuit to the proximal hub 220a (or proximal hub 320a) and the distal connector 420b can be configured for connecting the flexible circuit to the distal hub 220b. The arms 432 can further include one or more electrodes 26 that can be configured for mapping or ablation of tissue as previously described. As will be appreciated, the flexible circuit 428 can be formed using known methods of forming flexible circuits including, but not limited to, forming metallic layers of traces and bonded dielectric layers. The traces can be connected to the electrodes 26.

As shown in FIG. 4C, the flexible circuit 428 can include an electrode 26 on a first side of the arm 432 and a reference electrode 460 on a second side of the arm 432. The electrode 26 can be configured for mapping or ablation of tissue while the reference electrode 460 can be configured for receiving far field signals conducted through the blood or other fluid in an organ. As will be appreciated, the far field signals detected by the reference electrode 460 can be received and processed by a computer (e.g., the PIU 30) to compare the signals received from the electrodes 26 to the signals received from the reference electrodes 460 to ensure the signals received for mapping are representative of electrocardiograhic signals through the tissue.

The reference electrodes can be configured to measure the electrical signals from the fluid and/or blood directly adjacent to an electrode that is touching tissue. As such, the reference electrodes are insulated from touching tissue and the resulting signals collected are non-local far-field signals. The information from this reference electrode can be used to cancel out far-field signal from the adjacent, tissue touching electrode to ensure that the tissue touching electrode collects local information only.

As shown in FIG. 4D, the flexible circuit 428 can include termination points 42 disposed on the proximal connector 420a for electrically connecting the electrodes 26 to a computer, such as a PIU 30, or to an ablation energy generator 50. The termination points 42 can each be electrically connected by traces to an electrode 26.

As shown in FIG. 4A, each arm 432 of the flexible circuit 428 can be divided into several individual arms 432a-432e. For example, the flexible circuit 428 can include a first arm 432a connected to the proximal connector 420a at a first end and a second arm 432b at a second end. The second arm 432b can be configured to extend distally such that the second arm 432b can extend substantially along the entire edge of the cylindrical structure of the basket assembly 28. The second arm 432b can be connected to a third arm 432c distal the first arm 432a but proximal the distal connector 420b. The third arm 432c can be connected to the distal connector 420b at a distal end of the third arm 432c. As will be appreciated, in some examples, the first arm 432a, the second arm 432b, and the third arm 432c can be a single continuous arm extending between the proximal connector 420a and the distal connector 420b.

The flexible circuit 428 can further include a fourth arm 432d and a fifth arm 432e that can diverge from the first arm 432a at a point 433a where the first arm 432a and the second arm 432b are connected. The fourth arm 432d and the fifth arm 432e can extend along the length of the flexible circuit 428 distally and converge together at a point 433b where the second arm 432b and third arm 432c are attached to each other. As will be appreciated, all of the arms 432a-432e can be a continuous piece of biocompatible material or all of the arms 432a-432e can be individual arms formed from biocompatible material that are connected together to form the arms 432. In some examples, neighboring fourth arms 432d and fifth arms 432e can connect to each other at connection points 434 to connect the flexible circuit 428 together.

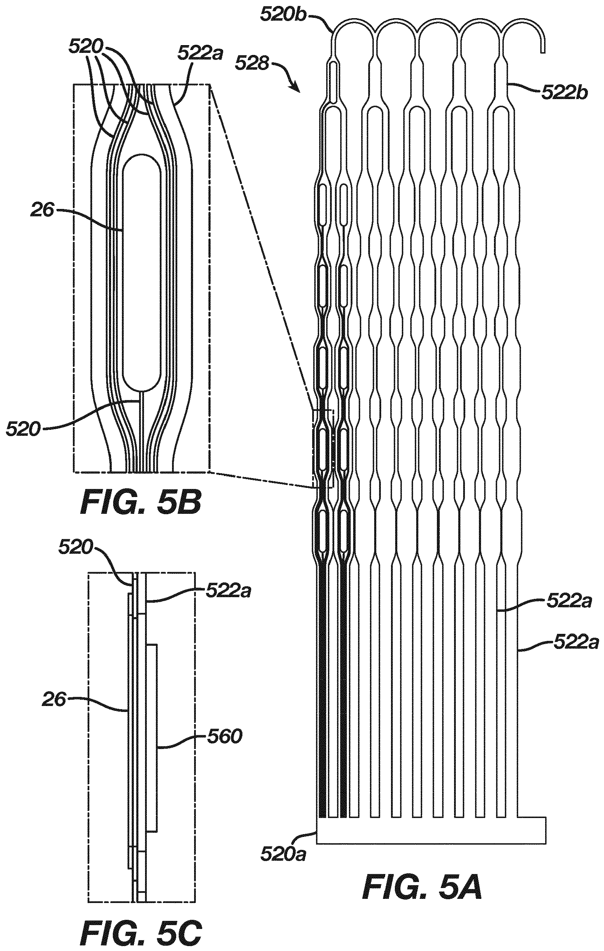

FIGs. 5A-5C show views of another example flexible circuit. FIG. 5A is a front view, FIG. 5B is a detail view, and FIG. 5C is a side detail view of a flexible circuit, in accordance with the disclosed technology. The flexible circuit 528 can be similar to the flexible circuit 428 just described. For example, the flexible circuit 528 can include a plurality of electrodes 26 and reference electrodes 560 that can include all of the features previously described in relation the electrodes 26 and the reference electrodes 460. Furthermore, the flexible circuit 528 can include traces 520 that can be electrically connected to the electrodes 26 and termination points (not shown). Furthermore, the flexible circuit 528 can include a proximal connector 520a for connecting the flexible circuit 528 to a proximal hub 220a and a distal connector 520b for connecting the flexible circuit 528 to a distal hub 220b as described previously.

The flexible circuit 528 can include a first arm 522a that can be attached to the proximal connector 520a at a first end and a second arm 522b at a second end. In this case, however, two first arms 522a can extend from the proximal connector 520a and converge together to attached to the second arm 522 proximate the distal connector 520b.

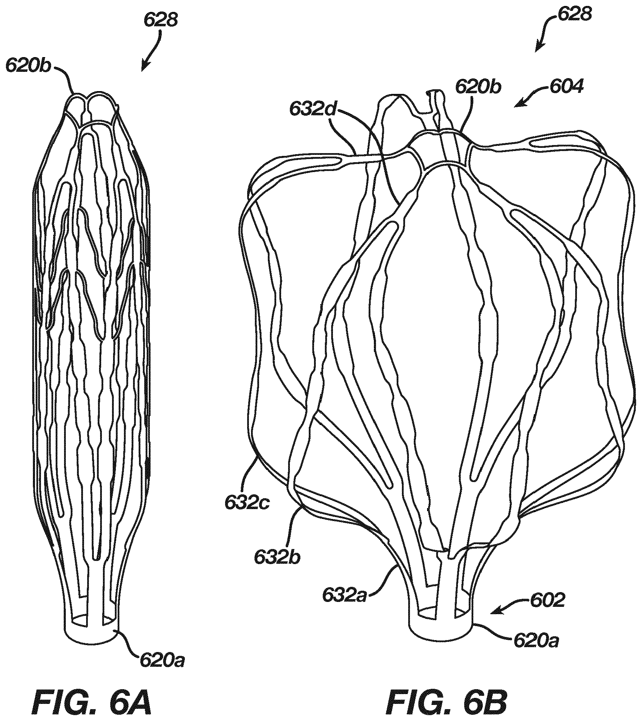

FIG. 6A illustrates a flexible circuit 628 in a collapsed configuration while FIG. 6B illustrates a flexible circuit 628 in an expanded configuration, in accordance with the disclosed technology. The flexible circuit 628 can be formed similar to the flexible circuit 428 and flexible circuit 528 and include electrodes 26 and reference electrodes 460, 560 as previously described. Furthermore, the flexible circuit 628 can include a proximal connector 620a at a proximal end 602 and a distal connector 620b and a distal end 604.

The flexible circuit 628 can include a first arm 632a connected to the proximal connector 620a at a first end and diverge into a second arm 632b and a third arm 632c at a second end. The second arm 632b and the third arm 632c can converge to a fourth arm 632d which can be connected to the distal connector 620b as shown. The second arm 632b and the third arm 632c can either converge together to connect to the same fourth arm 632, or the second arm 632b and the third arm 632c can converge toward a neighboring second arm 632b or neighboring third arm 632c, respectively, to connect with the respective neighboring second arm 632b or third arm 632c at different fourth arms 632d (as shown in FIGs. 6A and 6B).

As will be appreciated, the flexible circuit 428, 528, and 628 can be configured to align and connect with a basket assembly 28, 328. That is, the number of arms and configuration of arms can be made to match that of the spines such that the arms align with the spines. Furthermore, as will be appreciated, the flexible circuit 428, 528, or 628 can be made separate from and later attached to the basket assembly 28, 328 using any suitable attachment method. For example, the flexible circuit 428, 528, and 628 can be attached to the basket assembly 28, 328 using adhesive, heat treatment, fasteners, etc.

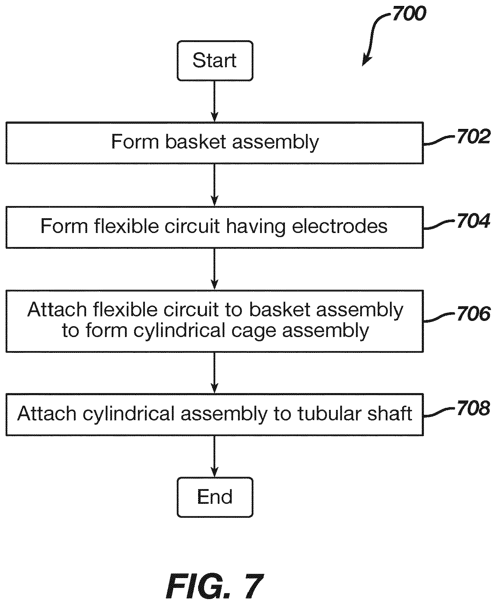

FIG. 7 is a flowchart of a method 700 of manufacturing a medical probe, in accordance with the disclosed technology. The method 700 can include forming 702 a basket assembly (e.g., basket assembly 28). Forming 702 the basket assembly, for example, can include forming a generally cylindrically-shaped basket with a plurality of spines as described herein. The method 700 can include forming a flexible circuit having electrodes (e.g., flexible circuit 428, 528, 628) (step 704) and attaching the flexible circuit to the basket assembly to form a generally cylindrical assembly (step 706). The method 700 can further include attaching the cylindrical assembly to a tubular shaft to form a generally cylindrically-shaped basket catheter (step 708). As will be appreciated, the method 700 just described can include any of the previous examples described herein.

The disclosed technology described herein can be further understood according to the following clauses:
Clause 1: A one-piece flex circuit comprising a proximal end and extending along a longitudinal axis to a distal end, the proximal end diverging into a plurality of first arms, each of the first arms dividing into second and third arms, each of the second and third arms connected to respective fourth arms proximate the distal end to define an elongated shape disposed about the longitudinal axis.
Clause 2: The one-piece flex circuit of Clause 1 further comprising a plurality of electrodes configured to ablate tissue of an organ of a patient.
Clause 3: The one-piece flex circuit of Clause 2, each electrode of the plurality of electrodes being attached to either the second arm or the third arm.
Clause 4: The one-piece flex circuit of Clauses 2 or 3, each electrode of the plurality of electrodes comprising a length ranging from about 0.2 millimeters to about 3 millimeters. In some examples, each electrode of the plurality of electrodes can comprise a length of about 1.3 millimeters
Clause 5: The one-piece flex circuit of any of Clauses 2-4, each electrode of the plurality of electrodes comprising a width ranging from about 0.2 millimeters to about 3 millimeters. In some examples, each electrode of the plurality of electrodes can comprise a width of about 0.25 millimeters.
Clause 6: The one-piece flex circuit of any of Clauses 1-5, further comprising a plurality of references electrodes.
Clause 7: The one-piece flex circuit of Clause 6, each reference electrode of the plurality of reference electrodes being disposed proximate an electrode of the plurality of electrodes.
Clause 8: The one-piece flex circuit of Clause 7, each electrode of the plurality of electrodes being disposed on an outwardly-facing surface of the one-piece flex circuit; and each reference electrode of the plurality of reference electrodes being disposed on an inwardly-facing surface of the one-piece flex circuit.
Clause 9: The one-piece flex circuit of any of Clauses 1-8, the one-piece flex circuit being configured for attachment to a basket catheter.
Clause 10: The one-piece flex circuit of Clause 9, the basket catheter comprising a generally cylindrical shape.
Clause 11: The one-piece flex circuit of any of Clauses 1-10, the one-piece flex circuit being configured to transition between an expanded form and a collapsed form.
Clause 12: A structural unit for an end effector comprising: a proximal hub; a distal hub positioned a distance along a longitudinal axis away from the proximal hub; and a plurality of spines extending between the proximal hub and the distal hub and forming a generally cylindrical structure, the plurality of spines comprising: a plurality of first members attached to the proximal hub, the plurality of first members diverging into a plurality of second and third members, each of the second and third members connected to respective fourth members proximate the distal hub.
Clause 13: The structural unit of Clause 12, each of the respective fourth members diverging into fifth and sixth members, each of the fifth and sixth members connected to the distal hub.
Clause 14: The structural unit of Clause 12, further comprising a fifth member extending between the first member and the fourth member.
Clause 15: The structural unit according to any of Clauses 12-14, the generally cylindrical structure configured to transition between a collapsed configuration and an expanded configuration.
Clause 16: The structural unit according to any of Clauses 12-15, the cylindrical structure comprising a substantially planar distal portion.
Clause 17: The structural unit according to any of Clauses 12-16, wherein the plurality of spines are formed from a planar sheet of biocompatible material.
Clause 18: The structural unit according to any of Clauses 12-16, wherein the plurality of spines are formed from a tube of biocompatible material.
Clause 19: The structural unit of Clauses 17 or 18, wherein the biocompatible material comprises nitinol.
Clause 20: A medical probe, comprising: a structural unit for an end effector, the structural unit comprising: a proximal hub; a distal hub positioned a distance along a longitudinal axis away from the proximal hub; and a plurality of spines extending between the proximal hub and the distal hub and forming a generally cylindrical structure, the plurality of spines comprising: a plurality of first members attached to the proximal hub, the plurality of first members diverging into a plurality of second and third members, each of the second and third members connected to respective fourth members proximate the distal hub; and a flexible circuit coupled to the structural unit, the flexible circuit comprising a proximal end and a distal end, the proximal end diverging into a plurality of first arms, each of the first arms dividing into second and third arms, each of the second and third arms connected to respective fourth arms proximate the distal end.
Clause 21: The medical probe of Clause 20, the cylindrical structure and the flexible circuit both being configured to transition together between a collapsed configuration and an expanded configuration.
Clause 22: The medical probe of Clause 20, each of the respective fourth members diverging into fifth and sixth members, each of the fifth and sixth members connected to the distal hub.
Clause 23: The medical probe of Clause 20, the plurality of spines further comprising a fifth member extending between the first member and the fourth member.
Clause 24: The medical probe of Clauses 20-23, the cylindrical structure comprising a substantially planar distal portion.
Clause 25: The medical probe of Clauses 20-24, the flexible circuit being formed separate from the structural unit and then attached to the structural unit to form the medical probe.
Clause 26: The medical probe of Clause 25, wherein the flexible circuit is bonded to the plurality of spines.
Clause 27: The medical probe of Clauses 20-26, wherein the flexible circuit is shaped substantially similar to the structural unit.
Clause 28: The medical probe according to any of Clauses 20-27, wherein the flexible circuit further comprises a plurality of electrodes.
Clause 29: The medical probe according to any of Clauses 20-28, the flexible circuit further comprising a plurality of electrical traces, each electrical trace of the plurality of electrical traces being in electrical communication with a respective electrode of the plurality of electrodes.
Clause 30: The medical probe according to any of Clauses 20-30, wherein the plurality of spines are formed from a planar sheet of biocompatible material.
Clause 31: The medical probe according to any of Clauses 20-30, wherein the plurality of spines are formed from a tube of biocompatible material.
Clause 32: The medical probe according to Clauses 30 or 31, wherein the biocompatible material comprises nitinol.
Clause 33: The medical probe according to any of Clauses 28-32, further comprising a reference electrode attached to the generally cylindrical structure.
Clause 34: The medical probe according to Clause 33, wherein the reference electrode is positioned proximate an electrode of the plurality of electrodes.
Clause 35: The medical probe according to Clause 34, further comprising a plurality of reference electrodes, each reference electrode of the plurality of reference electrodes being positioned proximate a respective electrode of the plurality of electrodes and attached to a respective spine of the plurality of spines on a side of the respective spine opposite each respective electrode of the plurality of electrodes.
Clause 36: The medical probe according to any of Clauses 20-35, further comprising an electromagnetic coil attached to the cylindrical structure, the electromagnetic coil configured to sense a magnetic field for position sensing.
Clause 37: The medical probe according to any of Clauses 20-36, wherein at least one of the plurality of electrodes is configured to map electrical signals in an organ of a patient.
Clause 38: The medical probe according to any of Clauses 20-37, wherein at least one electrode of the plurality of electrodes is configured to ablate tissue in an organ of a patient.
Clause 39: The medical probe according to Clause 38, wherein the one or more electrodes of the plurality of electrodes are configured to deliver electrical pulses for irreversible electroporation, the pulses having a peak voltage of at least 900 volts (V).
Clause 40: The medical probe according to any of Clauses 20-39, each first arm of the plurality of first arms being aligned with a first member of the plurality of first members.
Clause 41: The medical probe according to any of Clauses 20-40, each second arm being aligned with a second member.
Clause 42: The medical probe according to any of Clauses 20-41, each third arm being aligned with a third member.
Clause 43: The medical probe according to any of Clauses 20-42, each fourth arm being aligned with a fourth member.
Clause 44: A method of constructing a medical probe, the method comprising: forming a plurality of spines, the plurality of spines forming a substantially cylindrical shape, the plurality of spines comprising: a plurality of first members attached to a proximal hub, the plurality of first members diverging into a plurality of second and third members, each of the second and third members connected to respective fourth members proximate a distal hub; forming a flexible circuit including a plurality of electrodes; coupling the flexible circuit to the plurality of spines to form a cylindrical structure; and attaching the cylindrical structure to a distal end of a tubular shaft.
Clause 45: The method according to Clause 44, each of the respective fourth members diverging into fifth and sixth members, each of the fifth and sixth members connected to the distal hub.
Clause 46: The method according to Clause 44, the plurality of spines further comprising a fifth member extending between the first member and the fourth member.
Clause 47: The method according to any of Clauses 44-46, the flexible circuit further comprising a proximal end and a distal end, the proximal end diverging into a plurality of first arms, each of the first arms dividing into second and third arms, each of the second and third arms connected to respective fourth arms proximate the distal end.
Clause 48: The method according to any of Clauses 44-47, each first arm of the plurality of first arms being aligned with a first member of the plurality of first members.
Clause 49: The method according to any of Clauses 44-48, each second arm being aligned with a second member.
Clause 50: The method according to any of Clauses 44-49, each third arm being aligned with a third member.
Clause 51: The method according to any of Clauses 44-50, each fourth arm being aligned with a fourth member.
Clause 52: The method according to any of Clauses 44-51, wherein the plurality of spines comprises nitinol.
Clause 53: The method according to any of Clauses 44-52, the cylindrical structure being configured to transition between a collapsed configuration and an expanded configuration.
Clause 54: The method according to any of Clauses 44-53, the cylindrical structure comprising a substantially planar distal portion.
Clause 55: The method according to any of Clauses 44-54, wherein the flexible circuit is bonded to the plurality of spines.
Clause 56: The method according to any of Clauses 44-55 further comprising forming a plurality of electrical traces on the flexible circuit.
Clause 57: The method according to any of Clauses 44-56, wherein the flexible circuit is shaped substantially similar to the plurality of spines.
Clause 58: The method according to any of Clauses 44-57, further comprising attaching a reference electrode to the cylindrical structure.
Clause 59: The method according to Clause 58, further comprising positioning the reference electrode proximate an electrode of the plurality of electrodes.
Clause 60: The method according to Clause 59, further comprising attaching a plurality of reference electrodes to the cylindrical structure, each reference electrode of the plurality of reference electrodes being positioned proximate a respective electrode of the plurality of electrodes and attached to a respective spine of the plurality of spines on a side of the respective spine opposite each respective electrode of the plurality of electrodes.
Clause 61: The method according to any of Clauses 44-60 further comprising attaching an electromagnetic coil to the cylindrical structure, the electromagnetic coil configured to sense a magnetic field for position sensing.
Clause 62: The method according to any of Clauses 44-61, wherein at least one of the plurality of electrodes is configured to map electrical signals in an organ of a patient.
Clause 63: The method according to any of Clauses 44-62, wherein at least one electrode of the plurality of electrodes is configured to ablate tissue in an organ of a patient.
Clause 64: The method according to any of Clauses 44-63, wherein the at least one electrode of the plurality of electrodes is positioned at a distal end of the cylindrical structure.
Clause 65: The method according to any of Clauses 44-64, wherein the one or more electrodes of the plurality of electrodes are configured to deliver electrical pulses for irreversible electroporation, the pulses comprising a peak voltage of at least 900 volts (V).

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. A one-piece flex circuit comprising a proximal end and extending along a longitudinal axis to a distal end, the proximal end diverging into a plurality of first arms, each of the first arms dividing into second and third arms, each of the second and third arms connected to respective fourth arms proximate the distal end to define an elongated shape disposed about the longitudinal axis.

2. The one-piece flex circuit of claim 1, further comprising a plurality of electrodes configured to ablate tissue of an organ of a patient and a plurality of reference electrodes.

3. The one-piece flex circuit of claim 2, each electrode of the plurality of electrodes being disposed on an outwardly-facing surface of the one-piece flex circuit; and
each reference electrode of the plurality of reference electrodes being disposed on an inwardly-facing surface of the one-piece flex circuit.

4. The one-piece flex circuit of claim 1, the one-piece flex circuit being configured to transition between an expanded form and a collapsed form.

5. A medical probe, comprising:
a structural unit for an end effector, the structural unit comprising:
a proximal hub;
a distal hub positioned a distance along a longitudinal axis away from the proximal hub; and
a plurality of spines extending between the proximal hub and the distal hub and forming a generally cylindrical structure, the plurality of spines comprising:
a plurality of first members attached to the proximal hub, the plurality of first members diverging into a plurality of second and third members, each of the second and third members connected to respective fourth members proximate the distal hub; and
a flexible circuit coupled to the structural unit, the flexible circuit comprising a proximal end and a distal end, the proximal end diverging into a plurality of first arms, each of the first arms dividing into second and third arms, each of the second and third arms connected to respective fourth arms proximate the distal end.

6. The medical probe of claim 5, further comprising a plurality of electrodes configured to ablate tissue of an organ of a patient and a plurality of reference electrodes.

7. The medical probe of claim 6, the flexible circuit further comprising a plurality of electrical traces, each electrical trace of the plurality of electrical traces being in electrical communication with a respective electrode of the plurality of electrodes.

8. A method of constructing a medical probe, the method comprising:
forming a plurality of spines, the plurality of spines forming a substantially cylindrical shape, the plurality of spines comprising:
a plurality of first members attached to a proximal hub, the plurality of first members diverging into a plurality of second and third members, each of the second and third members connected to respective fourth members proximate a distal hub;
forming a flexible circuit including a plurality of electrodes;
coupling the flexible circuit to the plurality of spines to form a cylindrical structure; and
attaching the cylindrical structure to a distal end of a tubular shaft.

9. The medical probe according to claim 5 or the method according to claim 8, each of the respective fourth members diverging into fifth and sixth members, each of the fifth and sixth members connected to the distal hub.

10. The medical probe according to claim 5 or the method according to claim 8, the plurality of spines further comprising a fifth member extending between the first member and the fourth member.

11. The method according to claim 8 to 10, the flexible circuit further comprising a proximal end and a distal end, the proximal end diverging into a plurality of first arms, each of the first arms dividing into second and third arms, each of the second and third arms connected to respective fourth arms proximate the distal end.

12. The method according to claim 11, wherein:
each first arm of the plurality of first arms being aligned with a first member of the plurality of first members;
each second arm being aligned with a second member;
each third arm being aligned with a third member; and
each fourth arm being aligned with a fourth member.

13. The method according to claim 12, further comprising attaching a plurality of reference electrodes to the cylindrical structure, each reference electrode of the plurality of reference electrodes being positioned proximate a respective electrode of the plurality of electrodes and attached to a respective spine of the plurality of spines on a side of the respective spine opposite each respective electrode of the plurality of electrodes.

14. The medical probe of any of claims 5 to 7, 9 and 10, further comprising an electromagnetic coil attached to the cylindrical structure, the electromagnetic coil configured to sense a magnetic field for position sensing.

15. The medical probe of any of claims 5 to 7, 9, 10 and 14, each first arm of the plurality of first arms being aligned with a first member of the plurality of first members, and/or each second arm being aligned with a second member, and/or each third arm being aligned with a third member, and/or each fourth arm being aligned with a fourth member.
